(19) Europäisches Patentamt
European Patent Office
Office européen des brevets

(11) Veröffentlichungsnummer: **0 472 166 A1**

(12) **EUROPÄISCHE PATENTANMELDUNG**

(21) Anmeldenummer: **91113940.0**

(22) Anmeldetag: **20.08.91**

(51) Int. Cl.5: **C07D 471/04**, C07D 495/14, C07D 213/80, C07D 403/06, C07D 498/04, A61K 31/435, //(C07D471/04,221:00,209:00, 209:00),(C07D495/14,333:00, 221:00,209:00)

(30) Priorität: **21.08.90 CH 2700/90**

(43) Veröffentlichungstag der Anmeldung: **26.02.92 Patentblatt 92/09**

(84) Benannte Vertragsstaaten: **AT BE CH DE DK FR GB IT LI LU NL SE**

(71) Anmelder: **F. HOFFMANN-LA ROCHE AG**
**Postfach 3255**
**CH-4002 Basel(CH)**

(72) Erfinder: **Burner, Serge**
**rue de la Gendarmerie**
**F-68480 Durmenach-Ferrette(FR)**
Erfinder: **Widmer, Ulrich**
**Alleeweg 13**
**CH-4310 Rheinfelden(CH)**

(74) Vertreter: **Lederer, Franz, Dr. et al**
**Patentanwalt Dr. Franz Lederer**
**Lucile-Grahn-Strasse 22**
**W-8000 München 80(DE)**

(54) **Tricyclische Pyridonderivate.**

(57) Die neuen Pyridonderivate der allgemeinen Formel

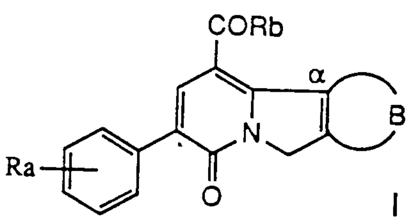

I

worin Ra Wasserstoff oder Halogen, Rb die Gruppe $-OR^1$ oder $-NR^2R^3$, $R^1$ eine unsubstituierte oder durch Hydroxy, niederes Alkoxy, Amino, niederes Alkylamino, di(niederes Alkyl)amino, di(niederes Alkyl)carbamoyl oder niederes Alkoxycarbonylamino substituierte niedere Alkylgruppe, $R^2$ Wasserstoff oder eine unsubstituierte oder durch Hydroxy, niederes Alkoxy, Amino, niederes Alkylamino, di(niederes Alkyl)amino, di(niederes Alkyl)-carbamoyl oder niederes Alkoxycarbonylamino substituierte niedere Alkylgruppe und $R^3$ Wasserstoff oder niederes Alkyl oder $R^2$ und $R^3$ zusammen mit dem Stickstoffatom eine unsubstituierte oder durch niederes Alkyl, Hydroxy, niederes Alkoxy, niederes Hydroxyalkyl, niederes Alkoxyalkyl oder Phenyl mono- oder disubstituierte 1-Azetidinyl-,1-Pyrrolidinyl-, 1-Piperidinyl-, 4-Morpholinyl-, 4-Thiomorpholinyl- oder 1-Piperazinylgruppe, B zusammen mit dem mit $\alpha$ bezeichneten Kohlenstoffatom die Gruppe $>C_\alpha$-S-CH = CH- oder $>C_\alpha$-$CR^4$ = CH-CH = CH- und $R^4$ Wasserstoff, Fluor oder Chlor bedeuten,
können zur Bekämpfung oder Verhütung von Krankheiten verwendet werden. Sie sind insbesondere muskelrelaxierend, sedativ-hypnotisch, anxiolytisch und/oder antikonvulsiv wirksam und können demnach bei der Bekämpfung oder Verhütung von Muskelspannungen, Spannungszuständen, Schlaflosigkeit, Angstzuständen und/oder

EP 0 472 166 A1

Konvulsionen verwendet werden.

Die vorliegende Erfindung betrifft neue tricyclische Pyridonderivate, insbesondere Verbindungen der allgemeinen Formel

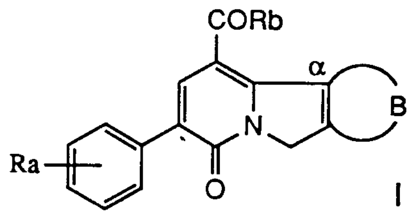

I

worin Ra Wasserstoff oder Halogen, Rb die Gruppe $-OR^1$ oder $-NR^2R^3$, $R^1$ eine unsubstituierte oder durch Hydroxy, niederes Alkoxy, Amino, niederes Alkylamino, di(niederes Alkyl)amino, di(niederes Alkyl)-carbamoyl oder niederes Alkoxycarbonylamino substituierte niedere Alkylgruppe, $R^2$ Wasserstoff oder eine unsubstituierte oder durch Hydroxy, niederes Alkoxy, Amino, niederes Alkylamino, di(niederes Alkyl)amino, di(niederes Alkyl)carbamoyl oder niederes Alkoxycarbonylamino substituierte niedere Alkylgruppe und $R^3$ Wasserstoff oder niederes Alkyl oder $R^2$ und $R^3$ zusammen mit dem Stickstoffatom eine unsubstituierte oder durch niederes Alkyl, Hydroxy, niederes Alkoxy, niederes Hydroxyalkyl, niederes Alkoxyalkyl oder Phenyl mono- oder disubstituierte 1-Azetidinyl-,1-Pyrrolidinyl-, 1-Piperidinyl-, 4-Morpholinyl-, 4-Thiomorpholinyl- oder 1-Piperazinylgruppe, B zusammen mit dem mit $\alpha$ bezeichneten Kohlenstoffatom die Gruppe $>C_\alpha$-S-CH = CH- oder $>C_\alpha-CR^4$ = CH-CH = CH- und $R^4$ Wasserstoff, Fluor oder Chlor bedeuten,
und pharmazeutisch annehmbare Säureadditionssalze von Verbindungen der Formel I mit einem basischen Substituenten.

Diese tricyclischen Pyridonderivate besitzen wertvolle pharmakologische Eigenschaften und können zur Bekämpfung oder Verhütung von Krankheiten verwendet werden. Sie sind insbesondere muskelrelaxierend, sedativ-hypnotisch, anxiolytisch und/oder antikonvulsiv wirksam und können demnach bei der Bekämpfung oder Verhütung von Muskelspannungen, Spannungszuständen, Schlaflosigkeit, Angstzuständen und/oder Konvulsionen verwendet werden.

Gegenstand der vorliegenden Erfindung sind: Die obigen Verbindungen der Formel I und die erwähnten Salze davon als solche; ein Verfahren und Zwischenprodukte zu deren Herstellung; die obigen Verbindungen der Formel I und die erwähnten Salze davon zur Anwendung als therapeutische Wirkstoffe; Arzneimittel auf der Basis dieser neuen Wirkstoffe und deren Herstellung; die Verwendung dieser Wirkstoffe bei der Bekämpfung oder Verhütung von Krankheiten; sowie deren Verwendung zur Herstellung von muskelrelaxierend, sedativ-hypnotisch, anxiolytisch und/oder antikonvulsiv wirksamen Mitteln.

Der Ausdruck "nieder" bezeichnet Reste und Verbindungen mit höchstens sieben, vorzugsweise höchstens vier Kohlenstoffatomen. Der Ausdruck "Alkyl" bezeichnet geradkettige oder verzweigte, gesättigte Kohlenwasserstoffreste, wie Methyl, Aethyl, Propyl, Isopropyl und t-Butyl. Der Ausdruck "Alkoxy" bezeichnet über ein Sauerstoffatom gebundene Alkylgruppen, wie Methoxy und Aethoxy. Der Ausdruck "Hydroxyalkyl" bezeichnet durch Hydroxy substituierte Alkylgruppen, wie Hydroxymethyl und 2-Hydroxyäthyl. Der Ausdruck "Halogen" bezeichnet die vier Formen Fluor, Chlor, Brom und Jod.

Das Symbol Ra bedeutet vorzugsweise Wasserstoff.

In einer bevorzugten Ausführungsform bedeuten das Symbol Rb die Gruppe $-OR^1$ und das Symbol $R^1$ niederes Alkyl.

In einer weiteren bevorzugten Ausführungsform bedeuten das Symbol Rb die Gruppe $-NR^2R^3$ und die Symbole $R^2$ und $R^3$ je niederes Alkyl oder zusammen mit dem Stickstoffatom eine durch niederes Alkoxy oder niederes Alkoxyalkyl monosubstituierte 1-Azetidinyl oder 1-Pyrrolidinylgruppe.

Das Symbol B bedeutet zusammen mit dem mit $\alpha$ bezeichneten Kohlenstoffatom vorzugsweise die Gruppe $>C_\alpha$-S-CH = CH-, $>C_\alpha$-CH = CH-CH = CH- oder $>C_\alpha$-CCl = CH-CH = CH-.

Die nachfolgend aufgeführten Verbindungen sind besonders bevorzugte Vetreter der durch die Formel I definierten Stoffklasse:

N,N-Dimethyl-4,6-dihydro-4-oxo-3-phenylpyrido[2,1-a]isoindol-1-carboxamid,

1-[(4,6-Dihydro-4-oxo-3-phenylpyrido[2,1-a]isoindol-1-yl)carbonyl]-3-methoxyazetidin,

1-[(4,6-Dihydro-6-oxo-7-phenylthieno[2',3':3,4]pyrrolo[1,2-a]pyridin-9-yl)carbonyl]-3-methoxyazetidin,

(R)-1-[(4,6-Dihydro-6-oxo-7-phenylthieno[2',3':3,4]-pyrrolo[1,2-a]pyridin-9-yl)carbonyl]-2-(methoxymethyl)pyrrolidin,

(S)-1-[(4,6-Dihydro-6-oxo-7-phenylthieno[2',3':3,4]pyrrolo[1,2-a]pyridin-9-yl)carbonyl]-3-methoxypyrrolidin und

1-[(10-Chlor-4,6-dihydro-4-oxo-3-phenylpyrido[2,1-a]isoindol-1-yl)carbonyl]-3-methoxyazetidin.

Die Verbindungen der Formel I und die pharmazeutisch annehmbaren Säureadditionssalze von Verbindungen der Formel I mit einem basischen Substituenten können erfindungsgemäss hergestellt werden, indem man

a) eine Carbonsäure der allgemeinen Formel

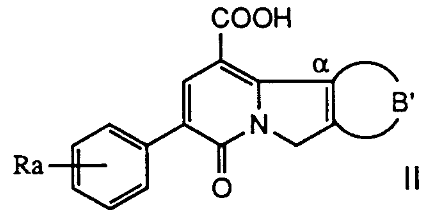

II

worin B' zusammen mit dem mit α bezeichneten Kohlenstoffatom die Gruppe $>C_\alpha$-S-CH = CH- oder $>C_\alpha$-CH = CH-CH = CH- bedeutet, und Ra die oben angegebene Bedeutung besitzt,
in Gegenwart eines Kondensationsmittels oder ein reaktives Derivat einer Carbonsäure der Formel II mit einer Verbindung der allgemeinen Formel

$R^1$-OH III

oder

$R^2R^3$NH IV

worin $R^1$, $R^2$ und $R^3$ die oben angegebene Bedeutung besitzen,
umsetzt, oder

b) eine Verbindung der allgemeinen Formel

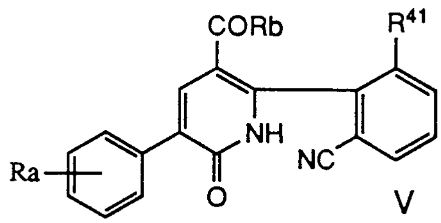

V

worin $R^{41}$ Chlor oder Fluor bedeutet, und Ra und Rb die oben angegebene Bedeutung besitzen,
mit Zink in Essigsäure behandelt, oder

c) eine Verbindung der allgemeinen Formel

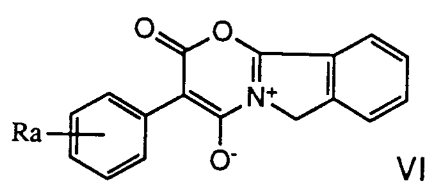

VI

worin Ra die oben angegebene Bedeutung besitzt, mit einer Verbindung der allgemeinen Formel

HC≡C-COOR$^{11}$ VII

worin $R^{11}$ niederes Alkyl bedeutet,
umsetzt, oder

d) in einer Verbindung der allgemeinen Formel

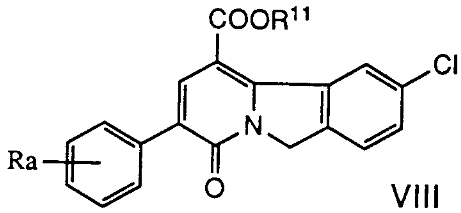

VIII

worin $R^{11}$ und Ra die oben angegebene Bedeutung besitzen,
das Chloratom durch Hydrogenolyse entfernt, oder
e) in einer Verbindung der allgemeinen Formel

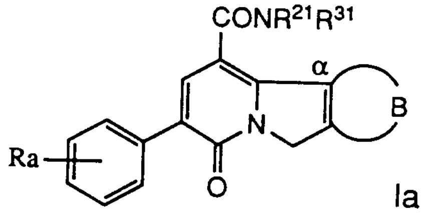

Ia

worin $R^{21}$ und $R^{31}$ zusammen mit dem Stickstoffatom eine durch niederes Alkoxy oder niederes Alkoxyalkyl substituierte 1-Azetidinyl-, 1-Pyrrolidinyl-, 1-Piperidinyl-, 4-Morpholinyl, 4-Thiomorpholinyl- oder 1-Piperazinylgruppe bedeuten, und Ra und B die oben angegebene Bedeutung besitzen,
die Aethergruppe spaltet, oder
f) in einer Verbindung der allgemeinen Formel

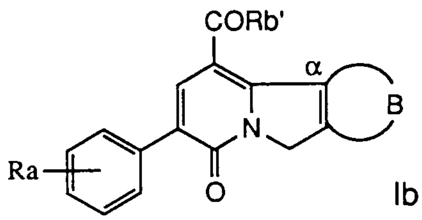

Ib

worin Rb' die Gruppe $-OR^{12}$ oder $-NR^{22}R^{32}$, $R^{12}$ und $R^{22}$ je eine durch niederes Alkoxycarbonylamino substituierte niedere Alkylgruppe und $R^{32}$ Wasserstoff oder niederes Alkyl bedeuten, und Ra und B die oben angegebene Bedeutung besitzen,
die niedere Alkoxycarbonylgruppe abspaltet, und
g) erwünschtenfalls eine erhaltene Verbindung der Formel I mit einem basischen Substituenten in ein pharmazeutisch annehmbares Säureadditionssalz überführt.

Gemäss Verfahrensvariante a) können Verbindungen der Formel I hergestellt werden, worin B zusammen mit dem mit $\alpha$ bezeichneten Kohlenstoffatom die Gruppe $>C_\alpha$-S-CH = CH- oder $>C_\alpha$-CH = CH-CH = CH-bedeutet. Die Reaktion kann beispielsweise in Gegenwart eines Kondensationsmittels und einer Base in einem inerten organischen Lösungsmittel durchgeführt werden. Geeignete Kondensationsmittel sind beispielsweise 2-(1H-Benzotriazol-1-yl) -1,1,3,3-tetramethyluronium hexafluorphosphat, 1H-1-Benzotriazolyloxy -tris(dimethylamino)phophonium hexafluorphosphat und dergleichen. Geeignete Lösungsmittel sind beispielsweise N,N-Dimethylformamid, Dioxan und dergleichen. Geeignete Basen sind beispielsweise tertiäre Amine, wie Triäthylamin, N-Methylmorpholin, 4-(Dimethylamino)pyridin und dergleichen. Die Reaktion wird vorzugsweise in einem Temperaturbereich von Raumtemperatur bis zur Rückflusstemperatur des Lösungsmittels durchgeführt.

Die gewünschte Reaktion kann jedoch auch dadurch bewerkstelligt werden, dass man die Carbonsäure der Formel II zunächst in an sich bekannter Weise in ein reaktives Derivat überführt und dieses dann in Gegenwart einer Base mit einer Verbindung der Formel III oder IV umsetzt. Als reaktive Derivate verwendet man vorzugsweise die entsprechenden Carbonsäurechloride, welche man vorzugsweise durch Behandeln mit Thionylchlorid in Gegenwart einer geringen Menge an N,N-Dimethylformamid in Toluol herstellt. Als

Basen eignen sich beispielsweise die bereits erwähnten tertiären Amine. Die Reaktion wird vorzugsweise in einem Temperaturbereich von etwa Raumtemperatur bis zur Rückflusstemperatur des Reaktionsgemisches durchgeführt, wobei man zweckmässigerweise bei Raumtemperatur arbeitet.

Gemäss Vefahrensvariante b) können Verbindungen der Formel I hergestellt werden, worin B zusammen mit dem mit α bezeichneten Kohlenstoffatom die Gruppe $>C_\alpha$-CCl=CH-CH=CH- oder $>C_\alpha$-CF=CH-CH=CH- bedeutet.
Das Zink wird vorzugsweise in Form von Zinkpulver eingesetzt, und die Reaktion wird vorzugsweise bei der Rückflusstemperatur des Reaktionsgemisches durchgeführt.

Gemäss Verfahrensvariante c) können Verbindungen der Formel I hergestellt werden, worin Rb die Gruppe $-OR^1$, $R^1$ niederes Alkyl und B zusammen mit dem mit α bezeichneten Kohlenstoffatom die Gruppe $>C_\alpha$-CH=CH-CH=CH bedeuten. Die Reaktion wird zweckmässigerweise bei erhöhter Temperatur durchgeführt, vorzugsweise oberhalb 80°C. Man verwendet daher vorzugsweise ein inertes Lösungsmittel, das bei erhöhter Temperatur, vorzugsweise oberhalb 80°C siedet. Besonders geeignete Lösungsmittel sind aromatische Kohlenwasserstoffe, wie Toluol oder Xylol oder dergleichen, wobei die Reaktion vorzugsweise bei der Rückflusstemperatur durchgeführt wird.

Gemäss Verfahrensvariante d) können ebenfalls Verbindungen der Formel I hergestellt werden, worin Rb die Gruppe $-OR^1$, $R^1$ niederes Alkyl und B zusammen mit dem mit α bezeichneten Kohlenstoffatom die Gruppe $>C_\alpha$-CH=CH-CH=CH- bedeuten. Für die Hydrogenolyse verwendet man als Katalysator vorzugsweise Palladiumkohle und als Lösungsmittel vorzugsweise einen niederen Alkohol, wie Methanol und Aethanol. Sie wird vorzugsweise in Gegenwart von Ammoniumformiat als Wasserstoffquelle und in einem Temperaturbereich von Raumtemperatur bis zur Rückflusstemperatur des Reaktionsgemisches durchgeführt.

Gemäss Verfahrensvariante e) können Verbindungen der Formel I hergestellt werden, worin Rb die Gruppe $-NR^2R^3$ und $R^2$ und $R^3$ zusammen mit dem Stickstoffatom eine durch Hydroxy oder niederes Hydroxyalkyl substituierte 1-Azetidinyl-, 1-Pyrrolidinyl-, 1-Piperidinyl-, 4-Morpholinyl-, 4-Thiomorpholinyl- oder 1-Piperazinylgruppe bedeuten. Diese Aetherspaltung wird vorzugsweise in einem inerten Lösungsmittel und in Gegenwart eines Alkalimetalljodides, wie Natriumjodid, und eines Tri(niederen Alkyl)chlorsilanes, wie Trimethylchlorsilan durchgeführt. Besonders geeignete Lösungsmittel sind Acetonitril und dergleichen. Die Reaktionstemperatur liegt vorzugsweise in einem Temperaturbereich von Raumtemperatur bis zur Rückflusstemperatur des Reaktionsgemisches, insbesondere bei der Rückflusstemperatur des Reaktionsgemisches.

Gemäss Verfahrensvariante f) können Verbindungen der Formel I hergestellt werden, worin Rb die Gruppe $-OR^1$ oder $-NR^2R^3$, $R^1$ und $R^2$ je eine durch Amino substituierte niedere Alkylgruppe und $R^3$ Wasserstoff oder niederes Alkyl bedeuten. Diese Reaktion wird vorzugsweise durch Behandeln mit Trifluoressigsäure durchgeführt. Die Reaktionstemperatur liegt vorzugsweise in einem Temperaturbereich von Raumtemperatur bis zur Rückflusstemperatur des Reaktionsgemisches, wobei man vorzugsweise bei der Raumtemperatur arbeitet.

Gemäss Verfahrensvariante g) können Verbindungen der Formel I, welche einen basischen Substituenten besitzen, in pharmazeutisch annehmbare Säureadditionssalze übergeführt werden. Es kommen dabei sowohl Salze mit anorganischen als auch Salze mit organischen Säuren in Betracht. Beispiele derartiger Salze sind Hydrochloride, Hydrobromide, Sulfate, Nitrate, Citrate, Acetate, Maleate, Succinate, Methansulfonate, p-Toluolsulfonate und dergleichen. Diese Salze können nach an sich bekannten und jedem Fachmann geläufigen Methoden hergestellt werden.

Die verschiedenen als Ausgangsstoffe verwendeten Verbindungen können beispielsweise gemäss den nachfolgenden Reaktionsschemata I-VI und den jeweils daran anschliessenden Beschreibungen der verschiedenen Reaktionen hergestellt werden.

Reaktionsschema I

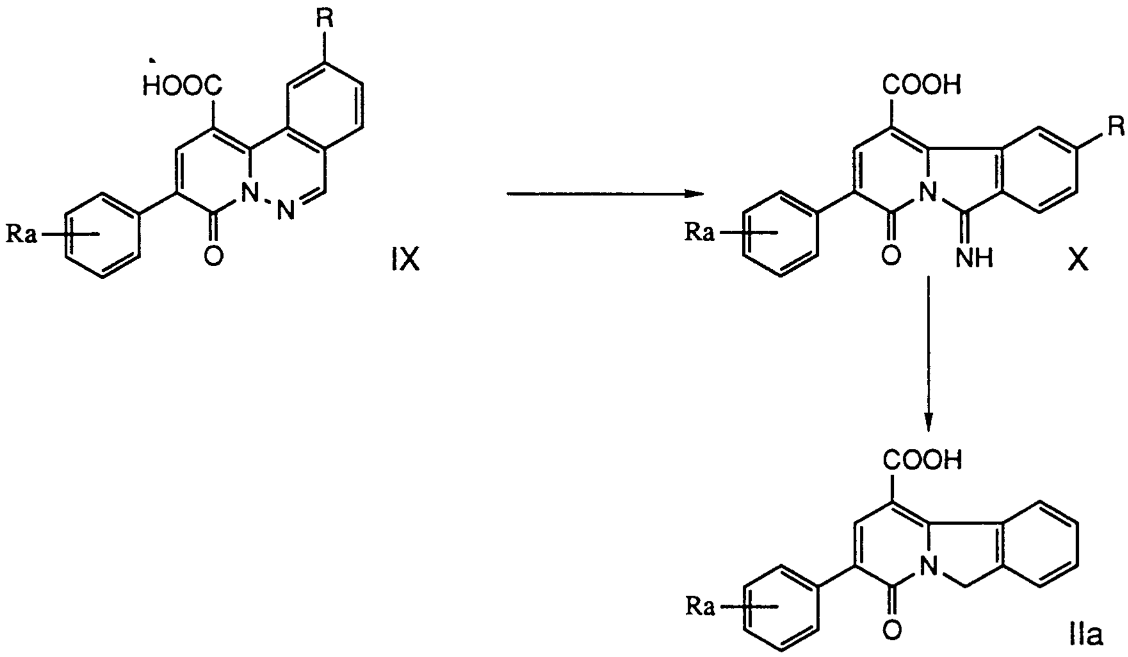

IX X IIa

R bedeutet Wasserstoff oder Chlor, und Ra besitzt die oben angegebene Bedeutung.

Durch Behandeln einer Verbindung der Formel IX mit einem Alkalimetallhydroxid, z.B. Kaliumhydroxid, in einem niederen Alkohol, wie Methanol und Aethanol, erhält man eine Verbindung der Formel X. Diese Reaktion wird vorzugsweise bei der Rückflusstemperatur des Reaktionsgemisches durchgeführt. Die Verbindungen der Formel IX gehören einer an sich bekannten Stoffklasse an. Derartige Verbindungen werden in der am 24. Juni 1987 publizierten Europäischen Patentpublikation Nr. 226,196 beschrieben.

Durch Hydrogenolyse einer Verbindung der Formel X erhält man die gewünschte Verbindung der Formel IIa, d.h. eine Verbindung der Formel II, worin B zusammen mit dem mit $\alpha$ bezeichneten Kohlenstoffatom die Gruppe $>C_{\alpha}$-CH=CH-CH=CH- bedeutet. Für die Hydrogenolyse verwendet man als Katalysator vorzugsweise Palladiumkohle, als Wasserstoffquelle vorzugsweise Ammoniumformiat und als Lösungsmittel vorzugsweise einen niederen Alkohol, wie Methanol und Aethanol. Sie wird vorzugsweise in einem Temperaturbereich von Raumtemperatur bis zur Rückflusstemperatur des Reaktionsgemisches durchgeführt.

Reaktionsschema II

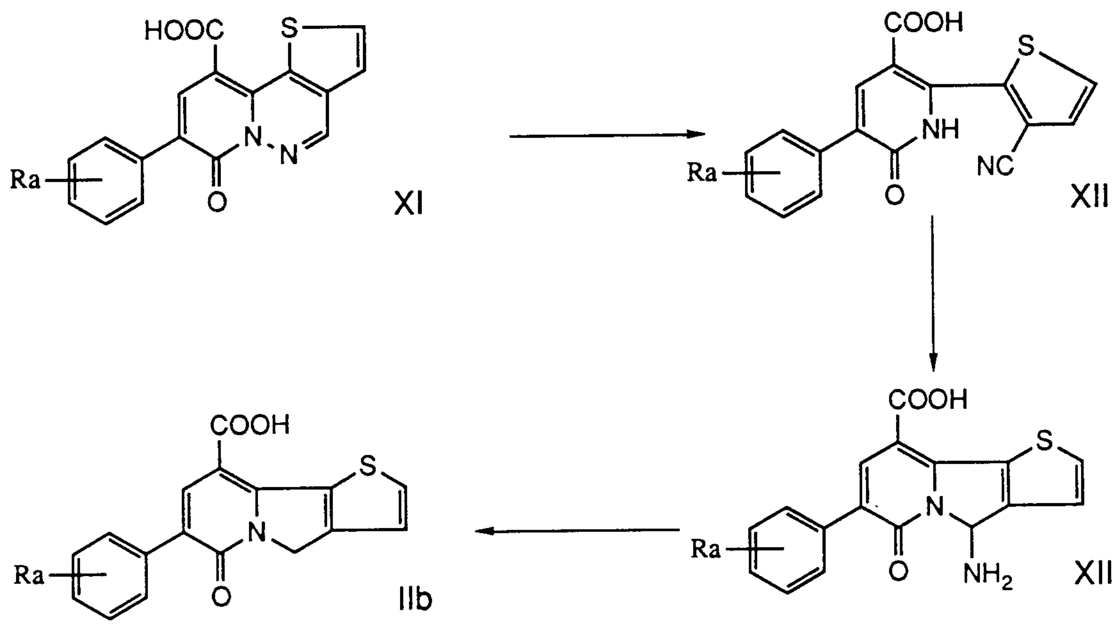

XI

XII

XIII

IIb

Ra besitzt die oben angegebene Bedeutung.

Durch Behandeln einer Verbindung der Formel XI mit einem Alkalimetallhydroxid, z.B. Kaliumhydroxid, in einem niederen Alkohol, wie Methanol und Aethanol, erhält man eine Verbindung der Formel XII. Diese Reaktion wird vorzugsweise bei der Rückflusstemperatur des Reaktionsgemisches durchgeführt. Die Verbindungen der Formel XI gehören ebenfalls einer an sich bekannten Stoffklasse an. Sie werden ebenfalls in der am 24. Juni 1987 publizierten Europäischen Patentpublikation Nr. 226,196 beschrieben.

Durch katalytische Hydrierung einer Verbindung der Formel XII erhält man eine Verbindung der Formel XIII. Für die katalytische Hydrierung verwendet man als Katalysator vorzugsweise Palladiumkohle und als Lösungsmittel vorzugsweise ein Gemisch aus einem niederen Alkohol, wie Methanol und Aethanol, und einer wässrigen Lösung eines Alkalimetallbicarbonates, wie Natriumbicarbonat. Sie wird vorzugsweise in einem Temperaturbereich von Raumtemperatur bis zur Rückflusstemperatur des Reaktionsgemisches durchgeführt.

Durch Behandeln einer Verbindung der Formel XIII mit Zink, vorzugsweise in Form von Zinkpulver, in Essigsäure, vorzugsweise bei der Rückflusstemperatur des Reaktionsgemisches, erhält man die gewünschte Verbindung der Formel IIb, d.h. eine Verbindung der Formel II, worin B zusammen mit dem mit $\alpha$ bezeichneten Kohlenstoffatom die Gruppe $>C_{\alpha}$-S-CH = CH- bedeutet.

<u>Reaktionsschema III</u>

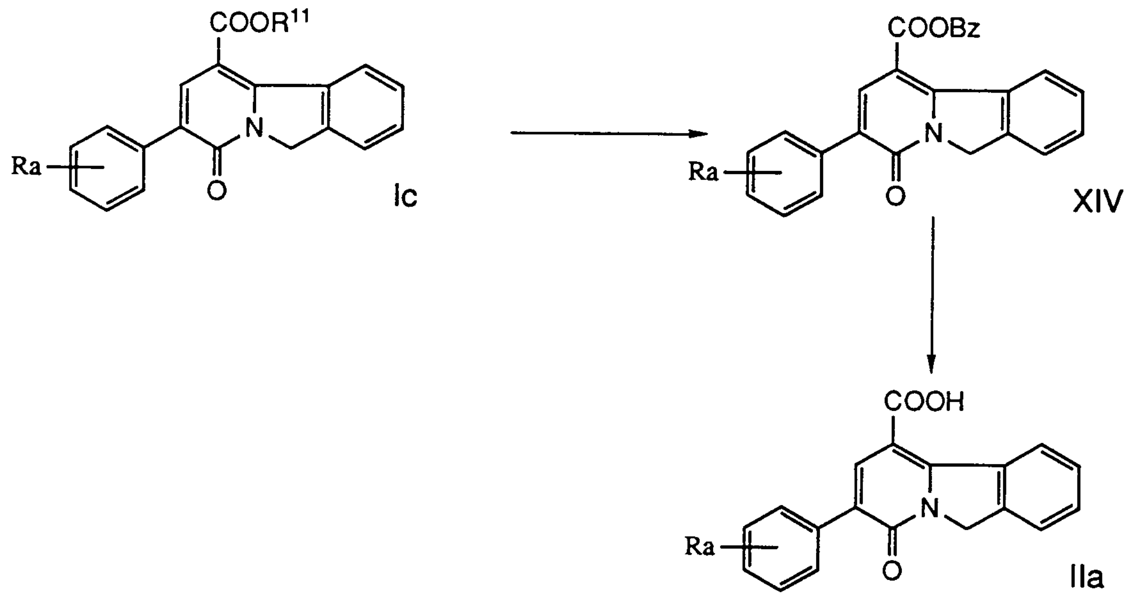

Ic

XIV

IIa

Bz bedeutet Benzyl, und $R^{11}$ und Ra besitzen die oben angegebene Bedeutung.

Durch Behandeln einer Verbindung der Formel Ic mit Benzylalkohol in Gegenwart von Tetraäthylorthotitanat erhält man die Verbindung der Formel XIV. Man verwendet als Lösungsmittel vorzugsweise Benzylalkohol und arbeitet zweckmässigerweise bei erhöhter Temperatur, vorzugsweise in einem Temperaturbereich von etwa 100 bis etwa 150° C.

Durch Hydrogenolyse einer Verbindung der Formel XIV erhält man die gewünschte Verbindung der Formel IIa, d.h. eine Verbindung der Formel II, worin B zusammen mit dem mit $\alpha$ bezeichneten Kohlenstoffatom die Gruppe $>C_{\alpha}$-CH = CH-CH = CH- bedeutet. Für die Hydrogenolyse verwendet man als Katalysator vorzugsweise Palladiumkohle, als Wasserstoffquelle elementaren Wasserstoff und als Lösungsmittel vorzugsweise einen niederen Alkohol, wie Methanol und Aethanol. Sie wird vorzugsweise in einem Temperaturbereich von Raumtemperatur bis zur Rückflusstemperatur des Reaktiongemisches, zweckmässigerweise jedoch bei Raumtemperatur durchgeführt.

Reaktionsschema IV

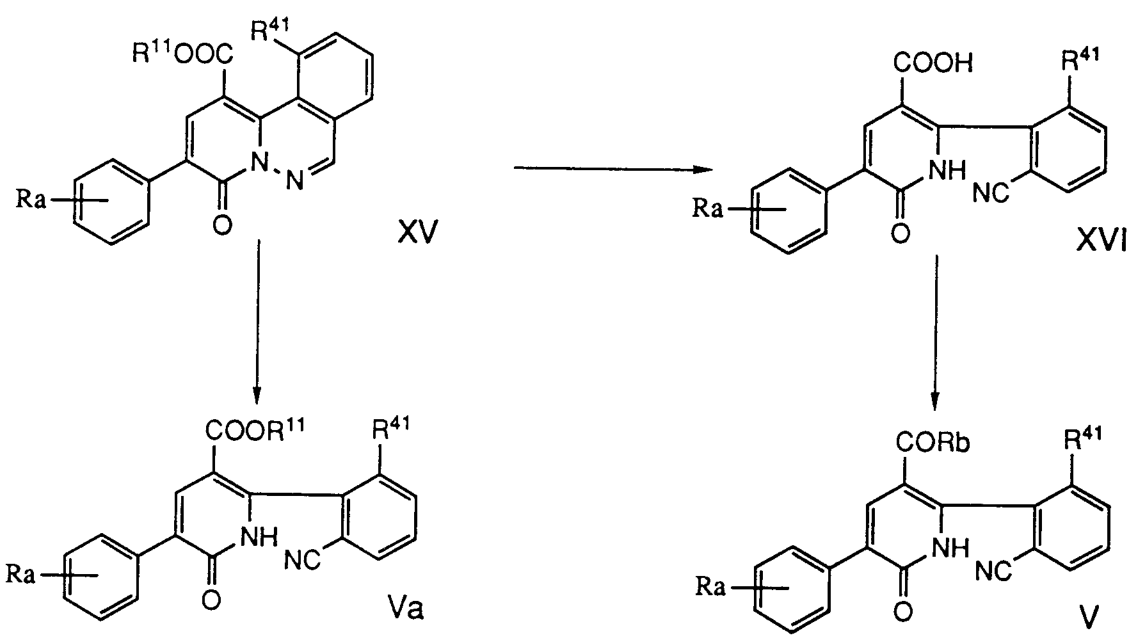

XV

XVI

Va

V

Ra, $R^{11}$ und $R^{41}$ besitzen die oben angegebene Bedeutung.

Durch Behandeln einer Verbindung der Formel XV mit einem Alkalimetallhydroxid, z.B. Kaliumhydroxid, in einem niederen Alkohol, wie Methanol und Aethanol, erhält man eine Verbindung der Formel XVI. Diese Reaktion wird vorzugsweise bei der Rückflusstemperatur des Reaktionsgemisches durchgeführt.

Durch Behandeln einer Verbindung der Formel XVI mit Thionylchlorid in Gegenwart einer geringen Menge an N,N-Dimethylformamid in Toluol erhält man das der Carbonsäure der Formel XVI entsprechende Carbonsäurechlorid, das man dann in Gegenwart einer Base mit einer Verbindung der obigen Formel III oder IV umsetzt. Man erhält dabei die gewünschre Verbindung der Formel V. Als Basen eignen sich dabei die bereits früher erwähnten tertiären Amine, insbesondere Triäthylamin. Geeignete Lösungsmittel sind beispielsweise N,N-Dimethylformamid und Dioxan. Die Reaktion wird zweckmässigerweise bei Raumtemperatur durchgeführt.

Durch Behandeln einer Verbindung der Formel XV mit einem niederen Alkalimetallalkoholat, vorzugsweise einem dem Rest $R^{11}$ entsprechenden Alkoholat, z.B. Natriummethylat, in einem niederen Alkohol, wie Methanol, erhält man die gewünschte Verbindung der Formel Va, d.h. eine Verbindung der Formel V, worin Rb die Gruppe $-OR^1$ und $R^1$ niederes Alkyl bedeuten. Diese Reaktion wird vorzugsweise bei der Rückflusstemperatur des Reaktionsgemisches durchgeführt. Die Verbindungen der Formel XV gehören einer an sich bekannten Stoffklasse an. Sie werden ebenfalls in der am 24. Juni 1987 publizierten Europäischen Patentpublikation Nr. 226,196 beschrieben.

Reaktionsschema V

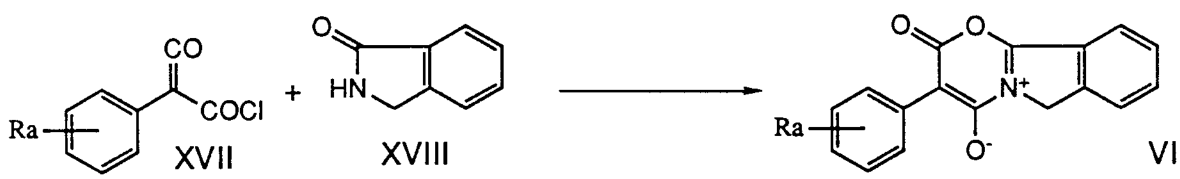

XVII XVIII VI

Ra besitzt die oben angegebene Bedeutung.

Durch Umsetzen einer Verbindung der Formel XVII mit einer Verbindung der Formel XVIII in einem inerten Lösungsmittel und in einem Temperaturbereich von etwa -10 °C bis etwa Raumtemperatur erhält man die gewünschte Verbindung der Formel VI. Als Lösungsmittel verwendet man vorzugsweise einen aromatischen Kohlenwasserstoff, wie Benzol oder Toluol. Die Reaktion wird vorzugsweise zwischen etwa 0° und 5 °C durchgeführt.

Reaktionsschema VI

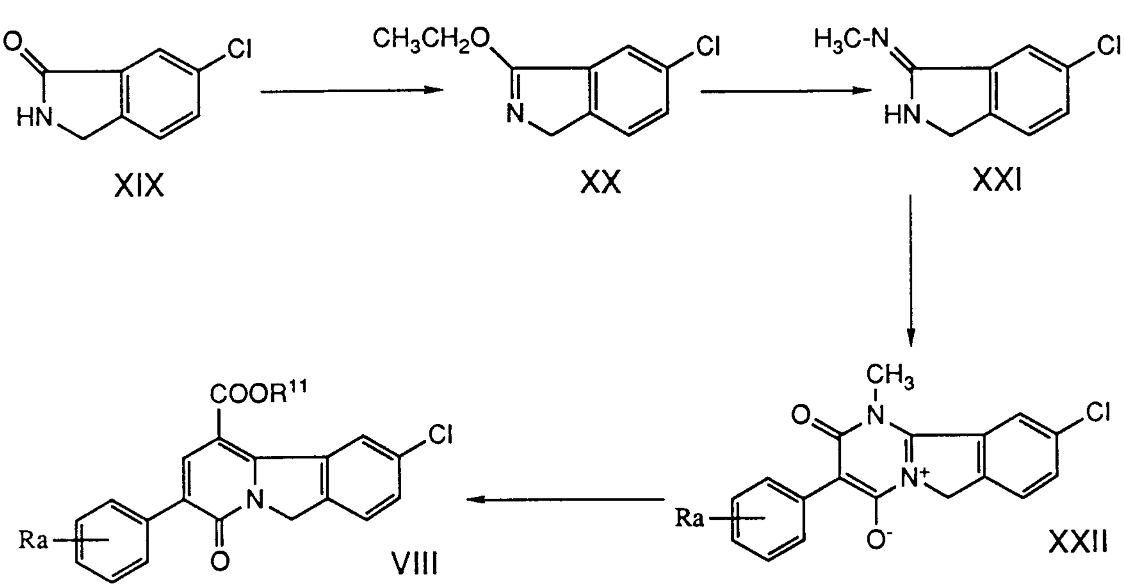

XIX XX XXI

XXII VIII

Ra und $R^{11}$ besitzen die oben angegebene Bedeutung.

Durch Behandeln einer Verbindung der Formel XIX mit Triäthyloxonium tetrafluoroborat erhält man eine Verbindung der Formel XX. Man verwendet als Lösungsmittel vorzugsweise einen chlorierten niederen Kohlenwasserstoff, wie Methylenchlorid, und arbeitet zweckmässigerweise bei Raumtemperatur.

Durch Behandeln der Verbindung der Formel XX mit Methylamin hydrochlorid erhält man das Hydrochlorid der Verbindung der Formel XXI, das man mit einer geeigneten Base, z.B. Natronlauge, in die Verbindung der Formel XXI überführen kann. Man verwendet als Lösungsmittel vorzugsweise einen niederen Alkohol, wie Methanol oder Aethanol, und arbeitet zweckmässigerweise bei der Rückflusstemperatur des Reaktionsgemisches.

Durch Umsetzen einer Verbindung der obigen Formel XVII mit der Verbindung der Formel XXI in einem inerten Lösungsmittel und in einem Temperaturbereich von etwa -10°C bis etwa Raumtemperatur erhält man eine Verbindung der Formel XXII. Als Lösungsmittel verwendet man vorzugsweise einen aromatischen Kohlenwasserstoff, wie Benzol oder Toluol. Die Reaktion wird vorzugsweise zwischen etwa 0° und 5°C durchgeführt.

Durch Umsetzen einer Verbindung der Formel XXII mit einer Verbindung der obigen Formel VII erhält man die gewünschte Verbindung der Formel VIII. Die Reaktion wird zweckmässigerweise bei erhöhter Temperatur durchgeführt, vorzugsweise oberhalb 80°C. Man verwendet daher vorzugsweise ein inertes Lösungsmittel, das bei erhöhter Temperatur, vorzugsweise oberhalb 80°C siedet. Besonders geeignete Lösungsmittel sind aromatische Kohlenwasserstoffe, wie Toluol oder Xylol oder dergleichen, wobei die Reaktion vorzugsweise bei der Rückflusstemperatur durchgeführt wird.

Die als Zwischenprodukte verwendeten Verbindungen der Formeln II, V, VI, VIII, X, XII, XIII, XIV, XVI und XXII sind neu und ebenfalls Gegenstand der vorliegenden Erfindung. Die übrigen als Ausgangsstoffe oder Zwischenprodukte verwendeten Verbindungen gehören an sich bekannten Stoffklassen an.

Wie bereits eingangs erwähnt besitzen die Verbindungen der Formel I wertvolle pharmakologische Eigenschaften. Sie entfalten insbesondere ausgeprägte muskelrelaxierende, sedativ-hypnotische, antikonvulsive und/oder anxiolytische Eigenschaften und weisen eine nur geringe Toxizität auf. Diese Eigenschaften können beispielsweise im nachfolgend beschriebenen und für die Erfassung derartiger Eigenschaften allgemein anerkannten Antipentetrazoltest nachgewiesen werden.

In diesem Tierversuch verabreicht man Ratten die zu testende Verbindung oral und 30 Minuten später 120 mg/kg Pentetrazol intraperitoneal, das in ungeschützten Versuchstieren 1-4 Minuten nach der Injektion Emprosthotonus und tonische Streckungen der vorderen und/oder hinteren Gliedmassen bewirkt. Man verwendet 10 Versuchstiere pro Dosis Testsubstanz. Nach Auszählen der geschützten Versuchstiere ermittelt man die $ED_{50}$ nach der Probit-Methode. Die $ED_{50}$ ist diejenige Dosis, welche 50% der Versuchstiere vor den durch Pentetrazol bewirkten krampfartigen Anfällen schützt. In der nachfolgenden Tabelle werden die Resultate zusammengestellt, welche mit repräsentativen Vertretern der durch die allgemeine Formel I definierten Verbindungsklasse in dem vorstehend geschilderten Versuch erhalten worden sind. Die Tabelle enthält ausserdem Angaben über die akute Toxizität ($DL_{50}$) dieser Verbindungen in mg/kg bei einmaliger oraler Verabreichung an Mäusen.

**Tabelle**

| Verbindung der Formel I, worin | | | $ED_{50}$ in mg/kg p.o. | $DL_{50}$ in mg/kg p.o. |
|---|---|---|---|---|
| Ra | Rb | B | | |
| H | $Me_2N$- | -CH=CH-CH=CH- | 0,73 | 375 |
| " | 3-MeO-Azetidino | " | 0,39 | - |
| " | " | -S-CH=CH- | 2,1 | 2500 |
| " | (R)-2-$MeOCH_2$-Py- | " | 0,76 | 625 |
| " | 3-MeO-Pyrrolidino | " | 1,7 | 375 |
| " | 3-MeO-Azetidino | -CCl=CH-CH=CH- | 2,2 | - |
| " | (R)-2-$MeOCH_2$-Py- | -CH=CH-CH=CH- | 2,3 | 1500 |
| " | 4-Me-Piperazino | " | 5,7 | 312 |
| " | Morpholino | -S-CH=CH- | 1,32 | 5000 |

Me = Methyl
Py = Pyrrolidino

Die Verbindungen der Formel I und die pharmazeutisch annehmbaren Säureadditionssalze von Verbindungen der Formel I mit einem basischen Substituenten können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidancien enthalten. Sie können auch noch andere therapeutisch wertvolle Stoffe enthalten. Arzneimittel enthaltend ein erfindungsgemässes Produkt und einen therapeutisch inerten Träger, sowie ein Verfahren zu deren Herstellung, das dadurch gekennzeichnet ist, dass man ein erfindungsgemässes Produkt und gegebenenfalls einen oder mehrere andere therapeutisch wertvolle Stoffe in eine galenische Darreichungsform bringt, sind ebenfalls Gegenstand der vorliegenden Erfindung.

Die erfindungsgemässen Produkte können, wie bereits erwähnt, bei der Bekämpfung oder Verhütung von Krankheiten, insbesondere bei der Bekämpfung von Konvulsionen und Angstzuständen, sowie für die Herstellung von Arzneimitteln mit muskelrelaxierenden, sedativ-hypnotischen, antikonvulsiven und/oder

anxiolytischen Eigenschaften verwendet werden. Die Dosierung kann dabei innerhalb weiter Grenzen variieren und ist natürlich in jedem einzelnen Fall den individuellen Gegebenheiten anzupassen. Bei oraler Verabreichung liegt die tägliche Dosis in einem Bereich von etwa 1 mg bis etwa 100 mg.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

Eine Suspension von 2,9 g 2,6-Dihydro-4-hydroxy-2-oxo -3-phenyl[1,3]oxazino[3,2-a]isoindol-5-ium-hydroxid (inneres Salz) in 100 ml Toluol wird unter Argon mit 4,2 ml Propiolsäuremethylester versetzt und das Reaktionsgemisch wird während 25 Stunden unter Rückfluss zum Sieden erhitzt. Man lässt dann abkühlen und entfernt das Lösungsmittel im Vakuum. Das kristalline Rohprodukt wird an Kieselgel mit Methylenchlorid chromatographiert und dann aus Essigester umkristallisiert. Man erhält 0,86 g Methyl 4,6-dihydro-4-oxo-3-phenyl-4H-pyrido[2,1-a]isoindol -1-carboxylat als weisse Kristalle mit einem Smp. von 206-207°.

Das als Ausgangsstoff verwendete 2,6-Dihydro-4-hydroxy-2-oxo -3-phenyl[1,3]oxazino[3,2-a]isoindol-5-ium-hydroxid (inneres Salz) kann wie folgt hergestellt werden:

Eine Lösung von 1,81 g α-Carbonyl-phenylacetylchlorid in 20 ml Benzol wird unter Argon im Eisbad gekühlt. Nach Zugabe von 1,33 g 2,3-Dihydro-1H-isoindol-1-on wird das Kühlbad entfernt, und die braune Suspension wird noch während 30 Minuten gerührt. Das Lösungsmittel wird im Vakuum abgedampft. Man erhält 2,9 g 2,6-Dihydro-4-hydroxy-2-oxo -3-phenyl[1,3]oxazino[3,2-a]isoindol-5-ium-hydroxid (inneres Salz) als gelbbraune Kristalle.

Beispiel 2

a) Eine Suspension von 1,29 g 4,6-Dihydro-4-oxo-3-phenylpyrido[2,1-a]isoindol-1-carbonsäure in 45 ml Toluol wird mit 0,2 ml N,N-Dimethylformamid und 1,85 ml Thionylchlorid versetzt und dann während 16 Stunden bei Raumtemperatur gerührt. Das Lösungsmittel wird im Vakuum entfernt, und der Rückstand wird in 40 ml Dioxan aufgenommen. Nacheinander werden nun 1,78 ml Triäthylamin und eine Lösung von 0,41 g 3-Methoxyazetidin in 3 ml Dioxan dazugegeben, worauf man während 30 Minuten bei 25° rührt. Nach Entfernen des Lösungsmittels im Vakuum wird der Rückstand in 100 ml Methylenchlorid gelöst. Diese Lösung wird mit 5-proz. Natriumhydrogencarbonatlösung und Wasser gewaschen, über Natriumsulfat getrocknet, filtriert und eingedampft. Durch Chromatographieren an Kieselgel mit Methylenchlorid/Diäthyläther (9:1) und Methylenchlorid/Aceton (9:1) und Umkristallisieren aus Aethanol erhält man 1,26 g 1-[(4,6-Dihydro--4-oxo-3-phenylpyrido[2,1-a]isoindol -1-yl)carbonyl]-3-methoxyazetidin als schwach gelbliche Kristalle mit einem Smp. von 174°.

In analoger Weise erhält man:

b) Mit Dimethylamin das N,N-Dimethyl-4,6-dihydro-4-oxo--3-phenylpyrido[2,1-a]isoindol -1-carboxamid mit einem Smp. von 207-209° (Aethanol).

c) Mit N-Aethyl-N-(2-methoxyäthyl)amin nach Chromatographieren an Kieselgel mit Diäthyläther/Toluol (2:1) das N-Aethyl-4,6-dihydro-N-(2-methoxyäthyl)-4-oxo -3-phenylpyrido[2,1-a]isoindol-1-carboxamid mit einem Smp. von 139-140° (Essigester).

d) Mit (R)-2-(Methoxymethyl)pyrrolidin nach Chromatographieren an Kieselgel mit Methylenchlorid/Diäthyläther (9:1 und 2:1) das (R)-1-[[4,6-Dihydro-4-oxo-3-phenylpyrido[2,1-a]isoindol -1-yl]carbonyl]-2-(methoxymethyl)pyrrolidin mit einem Smp. von 130-131° (Essigester).

e) Mit 2-Hydroxy-N,N-dimethylacetamid nach Chromatographieren an Kieselgel mit Methylenchlorid/Diäthyläther (9:1) und Methylenchlorid/Aceton (9:1) das (Dimethylcarbamoyl)methyl-4,6-dihydro-4-oxo -3-phenylpyrido[2,1-a]isoindol-1-carboxylat mit einem Smp. von 177-179° (Acetonitril).

Die als Ausgangsmaterial verwendete 4,6-Dihydro-4-oxo -3-phenylpyrido[2,1-a]isoindol-1-carbonsäure kann wie folgt hergestellt werden.

A. Eine Lösung von 1,62 g Tetraäthylorthotitanat in 71 ml Benzylalkohol wird mit 4,52 g Methyl 4,6-dihydro-4-oxo-3-phenylpyrido[2,1-a]isoindol -1-carboxylat versetzt und unter Argon während 3 Stunden bei 115-120° gerührt. Man lässt dann abkühlen, verrührt das Reaktionsgemisch während 1 Stunde mit 71 ml 1N Salzsäure, verdünnt mit 350 ml Wasser und extrahiert mehrmals mit Methylenchlorid. Die organischen Phasen werden mit gesättigter Natriumhydrogencarbonatlösung gewaschen, über Natriumsulfat getrocknet, filtriert und eingedampft. Man rührt den Rückstand während 1 Stunde in 710 ml Diäthyläther, saugt ab und trocknet das erhaltene Material im Vakuum. Man erhält 4,02 g Benzyl 4,6-dihydro-4-oxo-3-phenylpyrido[2,1-a]isoindol -1-carboxylat als gelbliche Kristalle mit einem Smp. von 229°.

B. Eine Suspension von 3,88 g Benzyl 4,6-dihydro-4-oxo-3-phenylpyrido[2,1-a]isoindol -1-carboxylat in 140 ml Aethanol wird mit 0,78 g 10-proz. Palladiumkohle versetzt, worauf man während 16 Stunden hydriert. Das Reaktionsgemisch wird mit 1400 ml Wasser verdünnt und mit 4,18 g Natriumcarbonat versetzt. Der Katalysator wird abfiltiert, und das Filtrat wird mit 25-proz. Salzsäure angesäuert. Die ausgefallenen Kristalle werden abgesaugt, mit Wasser gewaschen und im Vakuum getrocknet. Man erhält 2,85 g 4,6-Dihydro-4-oxo-3-phenylpyrido[2,1-a]isoindol-1-carbonsäure als weisse Kristalle mit einem Smp. von 295° (Zersetzung).

Beispiel 3

a) Eine Suspension von 2,0 g 4,6-Dihydro-4-oxo-3-phenylpyrido[2,1-a]isoindol-1-carbonsäure in 25 ml N,N-Dimethylformamid wird unter Argon mit 0,91 ml cis-2,6-Dimethylmorpholin und dann mit 0,81 ml N-Methylmorpholin und 2,8 g 2-(1H-Benzotriazol-1-yl) -1,1,3,3-tetramethyluronium hexafluorphosphat versetzt und noch während 4,5 Stunden bei Raumtemperatur gerührt. Das Reaktionsgemisch wird dann auf eine Mischung aus 50 ml gesättigter Natriumhydrogencarbonatlösung und 250 ml Wasser gegossen. Das auskristallisierte Rohprodukt wird abgesaugt und getrocknet. Nach Chromatographieren an Kieselgel mit Toluol und Toluol/Aceton (9:1) wird zweimal aus Essigester umkristallisiert. Man erhält 1,19 g cis-4-[-(4,6-Dihydro-4-oxo-3-phenylpyrido[2,1-a]isoindol -1-yl)carbonyl]-2,6-dimethylmorpholin als gelbe Kristalle mit einem Smp. von 226-228°.

In analoger Weise erhält man:

b) Mit Morpholin das 4-[(4,6-Dihydro-4-oxo-3-phenylpyrido[2,1-a]isoindol -1-yl)carbonyl]morpholin mit einem Smp. von 262-266° (Acetonitril).

c) Mit Diäthylamin das N,N-Diäthyl-4,6-dihydro-4-oxo-3-phenylpyrido[2,1-a]isoindol -1-carboxamid mit einem Smp. von 170-172° (Essigester).

d) Mit 3-Hydroxyazetidin das 1-[(4,6-Dihydro-4-oxo-3-phenylpyrido[2,1-a]isoindol -1-yl)carbonyl]-3-azetidinol mit einem Smp. von 238-242° (Methanol).

e) Mit 1-Phenylpiperazin das 1-[(4,6-Dihydro-4-oxo-3-phenylpyrido[2,1-a]isoindol -1-yl)carbonyl]-4-phenylpiperazin mit einem Smp. von 208-210° (Acetonitril).

f) Mit 1-Methylpiperazin nach anschliessender Behandlung mit ätherischer Salzsäure das 1-[(4,6-Dihydro-4-oxo-3-phenylpyrido[2,1-a]isoindol -1-yl)carbonyl]-4-methylpiperazin hydrochlorid mit einem Smp. von >300° (Methanol).

g) Mit tert.-Butyl-2-aminoäthyl-carbamat das tert.-Butyl-[2-(4,6-dihydro-4-oxo -3-phenylpyrido[2,1-a]-isoindol-1-carboxamido)äthyl]carbamat mit einem Smp. von 238-240° (Methanol/N,N-Dimethylformamid).

h) Mit Thiomorpholin das 4-[(4,6-Dihydro-4-oxo-3-phenylpyrido[2,1-a]isoindol -1-yl)carbonyl]tetrahydro-2H-1,4-thiazin mit einem Smp. von 266-268° (Methanol/N,N-Dimethylformamid).

i) Mit Ammoniak das 4,6-Dihydro-4-oxo-3-phenylpyrido[2,1-a]isoindol-1-carboxamid mit einem Smp. von 294-296° (Methanol/N,N-Dimethylformamid).

Die als Ausgangsmaterial verwendete 4,6-Dihydro-4-oxo-3-phenylpyrido[2,1-a]isoindol-1-carbonsäure kann wie folgt hergestellt werden:

A. 3,5 g 10-Chlor-4-oxo-3-phenyl-4H-pyrido[2,1-a]phthalazin -1-carbonsäure werden zu einer Lösung von 5,6 g Kaliumhydroxid in 60 ml Methanol gegeben, worauf man während 25 Stunden unter Rückfluss zum Sieden erhitzt. Das Lösungsmittel wird dann im Vakuum entfernt, und der Rückstand wird in 60 ml Wasser aufgenommen. Durch Zugabe von 2N Salzsäure wird das Produkt ausgefällt. Die Kristalle werden abgesaugt, mit Wasser gewaschen und im Vakuum getrocknet. Man erhält 3,32 g 9-Chlor-6-imino-4-oxo-3-phenyl-6H-pyrido [2,1-a]isoindol -1-carbonsäure als gelbliche Kristalle mit einem Smp. von 266-268° (Zersetzung).

B. Eine Suspension von 18,1 g 9-Chlor-6-imino-4-oxo-3-phenyl-6H-pyrido[2,1-a]isoindol -1-carbonsäure und 18 g 10-proz. Palladiumkohle in 900 ml Methanol wird mit 16,3 g Ammoniumformiat versetzt und dann während 90 Minuten unter Rückfluss zum Sieden erhitzt. Das Behandeln mit Ammoniumformiat und das anschliessende Erhitzen werden einmal wiederholt, worauf man den Katalysator abfiltriert, das Lösungsmittel im Vakuum entfernt und den Rückstand in 150 ml Wasser aufnimmt. Man säuert mit 1N Salzsäure an, saugt die Kristalle ab und wäscht sie mit Wasser. Man erhält 12,55 g 4,6-Dihydro-4-oxo-3-phenylpyrido[2,1-a]isoindol-1-carbonsäure mit einem Smp. von 277-278° (Zersetzung).

Diese Verbindung kann in analoger Weise auch ausgehend von 4-Oxo-3-phenyl-4H-pyrido[2,1-a]-phthalazin-1-carbonsäure hergestellt werden:

Man erhält zunächst die 6-Imino-4-oxo-3-phenyl-6H-pyrido[2,1-a]isoindol-1-carbonsäure mit einem Smp.

von 240-245° (Zersetzung) und daraus die 4,6-Dihydro-4-oxo-3-phenylpyrido[2,1-a]isoindol-1-carbonsäure mit einem Smp. von 276-278° (Zersetzung).

Beispiel 4

a) In Analogie zu Beispiel 2a) erhält man aus 2,2 g 4,6-Dihydro-6-oxo -7-phenylthieno[2',3':3,4]pyrrolo-[1,2-a]pyridin-9-carbonsäure und Diäthylamin 1,17 g 4,6-Dihydro-N,N-diäthyl-6-oxo -7-phenylthieno-[2',3':3,4]pyrrolo[1,2-a]pyridin-9-carboxamid mit einem Smp. von 182-183° (Toluol).

In analoger Weise erhält man:

b) Mit Morpholin das 4-[(4,6-Dihydro-6-oxo -7-phenylthieno[2',3':3,4]pyrrolo[1,2-a]pyridin -9-yl)carbonyl]-morpholin mit einem Smp. von 225-227° (Toluol).

c) Mit 3-Methoxyazetidin das 1-[(4,6-Dihydro-6-oxo -7-phenylthieno[2',3':3,4]pyrrolo[1,2-a]pyridin -9-yl)-carbonyl]-3-methoxyazetidin mit einem Smp. von 192-193° (Toluol).

d) Mit (R)-2-(Methoxymethyl)pyrrolidin das (R)-1-[(4,6-Dihydro-6-oxo -7-phenylthieno[2',3':3,4]pyrrolo[1,2-a]pyridin -9-yl)carbonyl]-2-(methoxymethyl)pyrrolidin mit einem Smp. von 150-152° (Toluol).

e) Mit Dimethylamin das 4,6-Dihydro-N,N-dimethyl-6-oxo -7-phenylthieno[2',3':3,4]pyrrolo[1,2-c]pyridin-9-carboxamid mit einem Smp. von 174-176° (Toluol).

f) Mit 3-Azetidinol das 1-[(4,6-Dihydro-6-oxo -7-phenylthieno[2',3':3,4]pyrrolo[1,2-c]pyridin -9-yl)carbonyl]-3-azetidinol mit einem Smp. von 269-271° (N,N-Dimethylformamid).

g) Mit (S)-2-(Methoxymethyl)pyrrolidin das (S)-1-[[4,6-Dihydro-6-oxo -7-phenylthieno[2',3':3,4]pyrrolo[1,2-a]pyridin -9-yl]carbonyl]-2-(methoxymethyl)pyrrolidin mit einem Smp. von 149-150° (Toluol).

h) Mit (S)-3-Methoxypyrrolidin das (S)-1-[(4,6-Dihydro-6-oxo -7-phenylthieno[2',3':3,4]pyrrolo[1,2-a]pyridin -9-yl)carbonyl]-3-methoxypyrrolidin mit einem Smp. von 159-162° (Toluol).

Die als Ausgangsmaterial verwendete 4,6-Dihydro-6-oxo -7-phenylthieno[2',3':3,4]pyrrolo[1,2-a]pyridin-9-carbonsäure kann wie folgt hergestellt werden:

A. Man gibt 48,7 g 7-Oxo-8-phenyl-7H-pyrido[1,2-b]thieno[2,3-d]pyridazin -10-carbonsäure zu einer methanolischen Kaliumhydroxidlösung (hergestellt aus 72 g Kaliumhydroxid und 640 ml Methanol) und erhitzt während etwa 24 Stunden unter Rückfluss zum Sieden. Die Reaktionsmischung wird im Vakuum etwa auf die Hälfte eingedampft, mit 800 ml Wasser versetzt und durch Zugabe von 1N Salzsäure auf pH 1 gestellt. Die ausgefallenen Kristalle werden abgesaugt, mit Wasser gewaschen und im Vakuum getrocknet. Man erhält 48,6 g 2-(3-Cyano-2-thienyl)-1,6-dihydro-6-oxo-5-phenylnicotinsäure als gelbe Kristalle mit einem Smp. von 255-257° (Methanol/N,N-Dimethylformamid).

B. Eine Suspension von 45,4 g 2-(3-Cyano-2-thienyl)-1,6-dihydro-6-oxo-5-phenylnicotinsäure in 850 ml Methanol wird mit einer Lösung von 59,2 g Natriumhydrogencarbonat in 280 ml Wasser versetzt, worauf man während 45 Minuten rührt. Dann werden 2,27 g 10-proz. Palladiumkohle dazugegeben und es wird bei Raumtemperatur hydriert. Man säuert nach beendeter Reaktion mit 400 ml 2N Salzsäure an, filtriert den Katalysator ab und engt im Vakuum ein. Nach Zugabe von 400 ml Wasser fallen Kristalle aus. Nach Rühren über Nacht wird abgesaugt, und die gelben Kristalle werden getrocknet. Man erhält in quantitativer Ausbeute 45,9 g 4-Amino-4,6-dihydro-6-oxo -7-phenylthieno[2',3':3,4]pyrrolo[1,2-a]pyridin-9-carbonsäure mit einem Smp. von 227-235°.

C. Eine Suspension von 45,7 g 4-Amino-4,6-dihydro-6-oxo -7-phenylthieno[2',3':3,4]pyrrolo[1,2-a]pyridin-9-carbonsäure in 915 ml Essigsäure wird mit 27,6 g Zinkpulver versetzt und während 2 Stunden unter Rückfluss zum Sieden erhitzt. Die Suspension wird dann abgekühlt und auf 3000 ml Wasser gegossen. Das überschüssige Zink wird grösstenteils durch Abdekantieren entfernt. Die ausgefallenen Kristalle werden abgesaugt und im Vakuum getrocknet. Man erhält 42,4 g 4,6-Dihydro-6-oxo -7-phenylthieno-[2',3':3,4]pyrrolo[1,2-a]pyridin-9-carbonsäure mit einem Smp. von >300° (Reinheit 85-90%). Dieses Material kann durch Umkristallisieren aus Methanol/N,N-Dimethylformamid weiter gereinigt werden.

Beispiel 5

Eine Suspension von 9,0 g 4,6-Dihydro-4-oxo-3-phenylpyrido[2,1-a]isoindol-1-carbonsäure in 240 ml Dioxan wird nacheinander mit 6,7 ml 2-(Dimethylamino)äthanol, 15,7 g 1H-1-Benzotriazolyloxy-tris-(dimethylamino)phosphonium hexafluorphosphat und 362 mg 4-Dimethylaminopyridin versetzt, worauf man unter Argon während 80 Minuten unter Rückfluss zum Sieden erhitzt, mit 1,58 g 1-Benzotriazolyloxy-tris-(dimethylamino)phosphonium hexafluorphosphat versetzt und noch während 1 Stunde weiter erhitzt. Man lässt dann abkühlen und saugt die ausgefallenen Kristalle ab. Nach Verrühren mit 890 ml Methylenchlorid, 475 ml Wasser und 70 ml gesättigter Natriumhydrogencarbonatlösung während 90 Minuten wird die organische Phase abgetrennt und eingedampft. Der Rückstand wird in 100 ml Methanol gelöst, worauf man

mit ätherischer Salzsäure ansäuert, filtriert und das Produkt durch Kühlen im Eisbad ausfällt. Man erhält 2,9 g 2-(Dimethylamino)äthyl 4,6-dihydro-4-oxo -3-phenylpyrido[2,1-a]isoindol-1-carboxylat als gelbliche Kristalle mit einem Smp. von 225-228°.

Beispiel 6

Eine Suspension von 0,9 g tert.-Butyl-[2-(4,6-dihydro-4-oxo -3-phenylpyrido[2,1-a]isoindol-1-carboxamido)äthyl]carbamat in 1,6 ml Trifluoressigsäure wird bei Raumtemperatur gerührt. Nach etwa 15 Minuten ist die Reaktion beendet, und das Reaktionsgemisch wird langsam mit 36 ml 0,25N Natronlauge versetzt, wobei das Produkt auskristallisiert. Es wird für eine kurze Zeit gerührt und dann abgesaugt. Nach Chromatographieren an Kieselgel mit Methylenchlorid/Methanol (9:1 und 2:1) und Umkristallisieren aus Methanol erhält man 0,31 g N-(2-Aminoäthyl)-4,6-dihydro-4-oxo -3-phenylpyrido[2,1-a]isoindol-1-carboxamid mit einem Smp. von 216-218°.

Beispiel 7

a) Eine Suspension von 1,85 g (R)-1-[[4,6-Dihydro-6-oxo -7-phenylthieno[2',3':3,4]pyrrolo[1,2-a]pyridin-9-yl]carbonyl] -2-(methoxymethyl)pyrrolidin in 20 ml Acetonitril wird unter Argon mit 2,74 g Natriumjodid versetzt, worauf man unter Rückfluss zum Sieden erhitzt und innerhalb von 25 Minuten 2,3 ml Trimethylchlorsilan dazutropft. Das Reaktionsgemisch wird während 45 Minuten bei der Rückflusstemperatur gehalten und dann auf eine aus 10 ml 0,1N Natriumthiosulfatlösung und 70 ml Wasser hergestellte Lösung gegossen. Die gelben Kristalle werden abgesaugt, an Kieselgel mit Methylenchlorid, Methylenchlorid/Diäthyläther (9:1), Methylenchlorid/Aceton (9:1) und Methylenchlorid/ Aceton (4:1) chromatographiert und dann aus Methanol umkristallisiert. Man erhält 0,67 g (R)-1-[[4,6-Dihydro-6-oxo-7-phenylthieno[2',3':3,4]pyrrolo[1,2-a]pyridin -9-yl]carbonyl]-2-pyrrolidinmethanol mit einem Smp. von 209-211°.

In analoger Weise erhält man:

b) Aus (R)-1-[[10-Chlor-4,6-dihydro-4-oxo -3-phenylpyrido[2,1-a]isoindol-1-yl]carbonyl] -2-(methoxymethyl)pyrrolidin das (R)-1-[[10-Chlor-4,6-dihydro-4-oxo -3-phenylpyrido[2,1-a]isoindol-1-yl]-carbonyl] -2-pyrrolidinmethanol mit einem Smp. von 158-160° (Acetonitril).

c) Aus (R)-1-[[10-Chlor-3-(m-chlorphenyl)-4,6-dihydro -4-oxopyrido[2,1-a]isoindol-1-yl]carbonyl] -2-(methoxymethyl)pyrrolidin das (R)-1-[[10-Chlor-3-(m-chlorphenyl)-4,6-dihydro -4-oxopyrido[2,1-a]isoindol-1-yl]carbonyl] -2-pyrrolidinmethanol mit einem Smp. von 228-230° (Acetonitril).

Beispiel 8

a) Ein Gemisch aus 1,17 g 1-[[2-(2-Chlor-6-cyanophenyl)-1,6-dihydro-6-oxo-5-phenyl -3-pyridyl]carbonyl]-3-methoxyazetidin, 0,91 g Zinkpulver und 25 ml Essigsäure wird während 15 Minuten unter Argon unter Rückfluss zum Sieden erhitzt. Das Reaktionsgemisch wird dann auf 125 ml Wasser gegossen, worauf die ausgefallenen Kristalle abgesaugt und mit Wasser gewaschen werden. Die wässrige Phase wird erschöpfend mit Methylenchlorid extrahiert. Die vereinigten organischen Phasen werden eingedampft, und der Rückstand wird mit den oben erhaltenen Kristallen vereinigt. Man chromatographiert dieses Material an Kieselgel mit Methylenchlorid/Aceton (9:1) und kristallisiert es dann aus Toluol um. Man erhält 0,33 g 1-[(10-Chlor-4,6-dihydro-4-oxo-3-phenylpyido[2,1-a]isoindol -1-yl)carbonyl]-3-methoxyazetidin als gelbe Kristalle mit einem Smp. von 235-238°.

In analoger Weise erhält man:

b) Aus 2-(2-Chlor-6-cyanophenyl)-1,6-dihydro-N,N-dimethyl-6-oxo -5-phenylnicotinamid das 10-Chlor-4,6-dihydro-N,N-dimethyl-4-oxo -3-phenylpyrido[2,1-a]isoindol-1-carboxamid mit einem Smp. von 213-216° (Acetonitril).

c) Aus 2-(2-Chlor-6-cyanophenyl)-1,6-dihydro-N,N-diäthyl-6-oxo -5-phenylnicotinamid das 10-Chlor-N,N-diäthyl-4,6-dihydro-4-oxo -3-phenylpyrido[2,1-a]isoindol-1-carboxamid mit einem Smp. von 180-182° (Essigester).

d) Aus 2-(2-Chlor-6-cyanophenyl)-1,6-dihydro-N-(2-methoxyäthyl) -6-oxo-5-phenylnicotinamid das 10-Chlor-4,6-dihydro-N-(2-methoxyäthyl)-4-oxo -3-phenylpyrido[2,1-a]isoindol-1-carboxamid mit einem Smp. von 251-257° (Methanol).

e) Aus 1-[[2-(2-Chlor-6-cyanophenyl)-1,6-dihydro-6-oxo-5-phenyl -3-pyridyl]carbonyl]-4-methylpiperazin nach anschliessender Behandlung mit äthanolischer Salzsäure das 4-[[10-Chlor-4,6-dihydro-4-oxo-3-phenylpyrido[2,1-a]isoindol -1-yl]carbonyl]-4-methylpiperazin hydrochlorid mit einem Smp. von >300°

(Wasser).

f) Aus 2-(2-Chlor-6-cyanophenyl)-N-äthyl-1,6-dihydro -N-(2-methoxyäthyl)-6-oxo-5-phenylnicotinamid das 10-Chlor-N-äthyl-N-(2-methoxyäthyl)-4-oxo -3-phenylpyrido[2,1-a]isoindol-1-carboxamid mit einem Smp. von 146-148° (Essigester).

g) Aus 1-[2-(2-Chlor-6-cyanophenyl-5-(m-chlorphenyl)-1,6-dihydro -6-oxo-nicotinoyl]-3-methoxyazetidin das 1-[[10-Chlor-3-(m-chlorphenyl)-4,6-dihydro -4-oxopyrido[2,1-a]isoindol-1-yl]carbonyl]-3-methoxyazetidin mit einem Smp. von 231-234° (Acetonitril).

h) Aus (R)-1-[2-(2-Chlor-6-cyanophenyl)-1,6-dihydro-6-oxo -5-phenylnicotinoyl]-2-(methoxymethyl)-pyrrolidin das (R)-1-[[10-Chlor-4,6-dihydro-4-oxo -3-phenylpyrido[2,1-a]isoindol-1-yl]carbonyl] -2-(methoxymethyl)pyrrolidin mit einem Smp. von 214-216° (Toluol).

i) Aus (R)-1-[2-(2-Chlor-6-cyanophenyl)-5-(m-chlorphenyl) -1,6-dihydro-6-oxo-nicotinoyl]-2-(methoxymethyl)pyrrolidin das (R)-1-[[10-Chlor-3-(m-chlorphenyl)-4,6-dihydro -4-oxopyrido[2,1-a]isoindol-1-yl]carbonyl] -2-(methoxymethyl)pyrrolidin mit einem Smp. von 136-138° (Essigester).

Die als Ausgangsmaterial verwendeten Verbindungen der Formel V können wie folgt hergestellt werden:

A.a) Ein Gemisch aus 72,9 g Methyl 11-chlor-4-oxo-3-phenyl-4H-pyrido[2,1-a]phthalazin -1-carboxylat, 112 g Kaliumhydroxid und 1200 ml Methanol wird unter Argon während 46 Stunden unter Rückfluss zum Sieden erhitzt. Man engt dann auf etwa 200 ml ein, verdünnt die gelbe Suspension mit 1000 ml Wasser, filtriert und extrahiert die wässrige Phase dreimal mit je 200 ml Methylenchlorid. Die wässrige Phase wird mit 25-proz. Salzsäure angesäuert, und die ausgefallenen Kristalle werden abfiltriert, mit Wasser gewaschen und im Vakuum getrocknet. Man erhält 69,7 g 2-[2-Chlor-6-cyanophenyl)-1,6-dihydro-6-oxo -5-phenylnicotinsäure als gelbliche Kristalle mit einem Smp. von 279-281° (Zersetzung).

In analoger Weise erhält man:

A.b) Aus Methyl-11-chlor-3-(m-chlorphenyl-4-oxo -4H-pyrido[2,1-a]phthalazin-1-carboxylat die 5-(m-Chlorphenyl)-2-(2-chlor-6-cyanophenyl) -1,6-dihydronicotinsäure mit einem Smp. von 308-311° (Zersetzung; N,N-Dimethylformamid/Methanol).

B.a) Ein Gemisch aus 1,4 g 2-(2-Chlor-6-cyanophenyl)-1,6-dihydro-6-oxo -5-phenylnicotinsäure, 40 ml Toluol und 0,1 ml N,N-Dimethylformamid wird mit 1,75 ml Thionylchlorid versetzt und während 2 Stunden unter Feuchtigkeitsausschluss gerührt. Das Lösungsmittel wird dann im Vakuum entfernt, und der Rückstand wird in 40 ml Dioxan aufgenommen. Nach Zugabe von 1,7 ml Triäthylamin wird das Reaktionsgemisch mit 0,38 g 3-Methoxyazetidin versetzt und während 30 Minuten gerührt. Nach dem Eindampfen wird der Rückstand mit 40 ml Wasser behandelt, worauf die gelben Kristalle abfiltriert und getrocknet werden. Man erhält 1,17 g 1-[[2-(2-Chlor-6-cyanophenyl)-1,6-dihydro-6-oxo-5-phenyl -3-pyridyl]carbonyl]-3-methoxyazetidin mit einem Smp. von 249-253°.

In analoger Weise erhält man:

B.b) Mit Dimethylamin das 2-(2-Chlor-6-cyanophenyl)-1,6-dihydro-N,N-dimethyl-6-oxo -5-phenylnicotinamid mit einem Smp. von 264-267° (Essigester).

B.c) Mit Diäthylamin das 2-(2-Chlor-6-cyanophenyl)-1,6-dihydro-N,N-diäthyl-6-oxo -5-phenylnicotinamid mit einem Smp. von 237-240° (Acetonitril).

B.d) Mit 2-Methoxyäthylamin das 2-(2-Chlor-6-cyanophenyl)-1,6-dihydro-N-(2-methoxyäthyl) -6-oxo-5-phenylnicotinamid mit einem Smp. von 252-255° (Methanol/N,N-Dimethylformamid).

B.e) Mit N-Methylpiperazin das 1-[[2-(2-Chlor-6-cyanophenyl)-1,6-dihydro-6-oxo-5-phenyl -3-pyridyl]-carbonyl]-4-methylpiperazin mit einem Smp. von 250-253° (Methanol).

B.f) Mit N-Aethyl-N-(2-methoxyäthyl)amin das 2-(2-Chlor-6-cyanophenyl)-N-äthyl-1,6-dihydro -N-(2-methoxyäthyl)-6-oxo-5-phenylnicotinamid mit einem Smp. von 184-186° (Essigester).

B.g) Mit (R)-2-(Methoxymethyl)pyrrolidin das (R)-1-[2-(2-Chlor-6-cyanophenyl)-1,6-dihydro-6-oxo -5-phenylnicotinoyl]-2-(methoxymethyl)pyrrolidin als Schaum.

In analoger Weise erhält man aus 5-(m-Chlorphenyl)-2-(2-chlor-6-cyanophenyl) -1,6-dihydronicotinsäure:

B.h) Mit 4-Methoxyazetidin das 1-[2-(2-Chlor-6-cyanophenyl)-5-(m-chlorphenyl)-1,6-dihydro -6-oxo-nicotinoyl]-3-methoxyazetidin mit einem Smp. von 239-243° (Acetonitril).

B.i) Mit (R)-2-(Methoxymethyl)pyrrolidin das (R)-1-[2-(2-Chlor-6-cyanophenyl)-5-(m-chlorphenyl) -1,6-dihydro-6-oxo-nicotinoyl]-2-(methoxymethyl)pyrrolidin als Schaum.

<u>Beispiel 9</u>

In Analogie zu Beispiel 3.B erhält man aus 2,4 g Methyl 9-chlor-4,6-dihydro-4-oxo-3-phenylpyrido[2,1-a]isoindol-1-carboxylat in Gegenwart von 2,4 g 10-proz. Palladiumkohle und insgesamt 3,6 g Ammoniumformiat 1,68 g Methyl 4,6-dihydro-4-oxo-3-phenylpyrido[2,1-a]isoindol -1-carboxylat mit einem Smp. von 198-202°.

Das als Ausgangsmaterial verwendete Methyl 9-chlor-4,6-dihydro-4-oxo-3-phenylpyrido[2,1-a]isoindol-1-carboxylat kann wie folgt hergestellt werden:

A. Eine Lösung von 151 g Triäthyloxonium tetrafluoroborat in 3200 ml Methylenchlorid unter Argon wird nach Zugabe von 106,6 g 6-Chlor-2,3-dihydro-1H-isoindol-1-on während 3,5 Stunden bei Raumtemperatur gerührt. Innerhalb von 40 Minuten versetzt man dann mit 1,28 1 gesättigter Natriumhydrogencarbonatlösung, trennt die organische Phase ab und wäscht sie mit 1,28 1 Wasser. Nach Trocknen über Natriumsulfat wird filtriert und eingedampft. Der Rückstand wird in 1,3 1 Diäthyläther aufgenommen, worauf man bei Raumtemperatur während etwa 30 Minuten rührt. Nach Entfernen von unlöslichem Material durch Filtration wird das Filtrat eingedampft. Man erhält 110,5 g 1-Aethoxy-6-chlor-3H-isoindol mit einem Smp. von 64-66°.

B. Eine Suspension von 110,5 g 1-Aethoxy-6-chlor-3H-isoindol und 41,9 g Methylamin hydrochlorid in 1100 ml Aethanol unter Argon wird während 4 Stunden unter Rückfluss zum Sieden erhitzt, worauf man auf 2° abkühlt, die ausgefallenen Kristalle absaugt und aus Aethanol umkristallisiert. Man erhält 91,0 g 6-Chlor-1-(methylimino)isoindolin hydrochlorid als weisse Kristalle mit einem Smp. von >250°.

C. Ein Gemisch aus 5,42 g 6-Chlor-1-(methylimino)isoindolin (hergestellt aus dem Hydrochlorid durch Behandeln mit 1,5 Aequivalenten 2N Natronlauge) und 120 ml Toluol wird unter Argon bei 0-5° innerhalb von 10 Minuten tropfenweise mit 5,96 g α-Carbonyl-phenylacetylchlorid versetzt. Man rührt während 30 Minuten, gibt anschliessend tropfenweise 4,47 g 1,5-Diazabicyclo[4.3.0]non-5-en in 30 ml Toluol dazu, rührt noch während 90 Minuten bei 0-5°, versetzt dann mit 50 ml Wasser und saugt die ausgefallenen Kristalle ab. Man wäscht dieses Material mit 35 ml Methylenchlorid und erhält nach dem Trocknen 3,66 g 9-Chlor-1,2-dihydro-4-hydroxy-1-methyl-2-oxo-3-phenyl -6H-pyrimido[2,1-a]isoindol-5-ium-hydroxid (inneres Salz) mit einem Smp. von 245° (Zersetzung). Dieses Produkt kann aus N,N-Dimethylformamid umkristallisiert werden und hat dann einen Smp. von 288°.

D. Ein Gemisch aus 333 mg 9-Chlor-1,2-dihydro-4-hydroxy-1-methyl-2-oxo-3-phenyl -6H-pyrimido[2,1-a]-isoindol-5-ium-hydroxid (inneres Salz) und 41 ml Toluol wird mit 0,86 ml Propiolsäure-methylester versetzt und während etwa 6,5 Tagen unter Rückfluss zum Sieden erhitzt, wobei alle 24 Stunden jeweils 0,86 ml Propiolsäure-methylester dazugegeben werden. Man kühlt das Reaktionsgemisch dann im Eisbad ab und saugt die ausgefallenen Kristalle ab. Man erhält nach Umkristallisieren aus N,N-Dimethylformamid 165 mg Methyl 9-chlor-4,6-dihydro-4-oxo -3-phenylpyrido[2,1-a]isoindol-1-carboxylat als gelbliche Kristalle mit einem Smp. von 269-273°.

Beispiel 10

a) Ein Gemisch aus 1,04 g Methyl 2-(2-fluor-6-cyanophenyl)-1,6-dihydro-6-oxo -5-phenylnicotinat, 0,98 g Zinkpulver und 30 ml Essigsäure wird während 30 Minuten unter Rückfluss zum Sieden erhitzt. Man filtriert heiss und dampft das Filtrat im Vakuum ein. Der Rückstand wird über Nacht in 30 ml Wasser gerührt, worauf die Kristalle abgesaugt und mit Methylenchlorid gewaschen werden. Durch Chromatographieren an Kieselgel mit Methylenchlorid/Diäthyläther (9:1) erhält man gelbe Kristalle, die aus Essigester umkristallisiert werden. Man erhält 0,23 g Methyl 10-fluor-4,6-dihydro-4-oxo -3-phenylpyrido[2,1-a]-isoindol-1-carboxylat mit einem Smp. von 134-136°.

In analoger Weise erhält man:

b) Aus 1,09 g Methyl 2-(2-chlor-6-cyanophenyl)-1,6-dihydro-6-oxo -5-phenylnicotinat 0,47 g Methyl 10-chlor-4,6--dihydro-4-oxo -3-phenylpyrido[2,1-a]isoindol-1-carboxylat mit einem Smp. von 172-174° (Essigester).

Die als Ausgangsstoffe verwendeten Verbindungen können in Analogie zu Beispiel 4.A wie folgt hergestellt werden:

A. Aus 2,1 g Methyl 1-fluor-4-oxo-3-phenyl-4H-pyrido[2,1-a]phthalazin -1-carboxylat erhält man mit 4 Aequivalenten Natriummethylat in Methanol nach Chromatographieren an Kieselgel mit Methylenchlorid/Diäthyläther (9:1) und Methylenchlorid/Aceton (9:1) 1,95 g Methyl 2-[2-fluor-6-cyanophenyl)-1,6-dihydro-6-oxo -5-phenylnicotinat als gelbe Kristalle mit einem Smp. von 228-233°.

B. Aus 5,5 g Methyl 11-chlor-4-oxo-3-phenyl-4H-pyrido[2,1-a]phthalazin -1-carboxylat erhält man 5,2 g Methyl 2-(2-chlor-6-cyanophenyl)-1,6-dihydro-6-oxo -5-phenylnicotinat mit einem Smp. von 247-250° (Methanol).

Beispiel A

Das N,N-Dimethyl-4,6-dihydro-4-oxo-3-phenylpyrido[2,1-a]isoindol -1-carboxamid kann in an sich bekannter Weise als Wirkstoff zur Herstellung von pharmazeutischen Präparaten nachfolgender Zusammensetzung verwendet werden:

| a) Tabletten | | mg/Tablette |
|---|---|---|
| Verbindung A | | 5 |
| Milchzucker | | 135 |
| Maisstärke | | 51 |
| Polyvinylpyrrolidon | | 8 |
| Magnesiumstearat | | 1 |
| | Tablettengewicht | 200 |

| b) Kapseln | | mg/Kapsel |
|---|---|---|
| Verbindung A | | 10 |
| Milchzucker | | 30 |
| Maisstärke | | 8,5 |
| Talk | | 1 |
| Magnesiumstearat | | 0,5 |
| | Kapselfüllgewicht | 50 |

**Patentansprüche**

**1.** Verbindungen der allgemeinen Formel

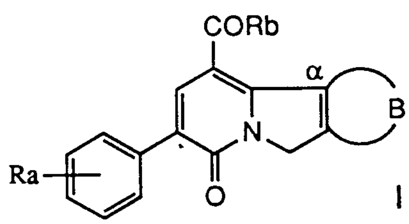

I

worin Ra Wasserstoff oder Halogen, Rb die Gruppe $-OR^1$ oder $-NR^2R^3$, $R^1$ eine unsubstituierte oder durch Hydroxy, niederes Alkoxy, Amino, niederes Alkylamino, di(niederes Alkyl)amino, di(niederes Alkyl)carbamoyl oder niederes Alkoxycarbonylamino substituierte niedere Alkylgruppe, $R^2$ Wasserstoff oder eine unsubstituierte oder durch Hydroxy, niederes Alkoxy, Amino, niederes Alkylamino, di-(niederes Alkyl)amino, di(niederes Alkyl)carbamoyl oder niederes Alkoxycarbonylamino substituierte niedere Alkylgruppe und $R^3$ Wasserstoff oder niederes Alkyl oder $R^2$ und $R^3$ zusammen mit dem Stickstoffatom eine unsubstituierte oder durch niederes Alkyl, Hydroxy, niederes Alkoxy, niederes Hydroxyalkyl, niederes Alkoxyalkyl oder Phenyl mono- oder disubstituierte 1-Azetidinyl-,1-Pyrrolidinyl-, 1-Piperidinyl-, 4-Morpholinyl-, 4-Thiomorpholinyl- oder 1-Piperazinylgruppe, B zusammen mit dem mit α bezeichneten Kohlenstoffatom die Gruppe $>C_\alpha$-S-CH=CH- oder $>C_\alpha-CR^4$=CH-CH=CH- und $R^4$ Wasserstoff, Fluor oder Chlor bedeuten,
und pharmazeutisch annehmbare Säureadditionssalze von Verbindungen der Formel I mit einem

basischen Substituenten.

**2.** Verbindungen nach Anspruch 1, worin Ra Wasserstoff bedeutet.

**3.** Verbindungen nach Anspruch 1 oder 2, worin Rb die Gruppe $-OR^1$ und $R^1$ niederes Alkyl bedeuten.

**4.** Verbindungen nach Anpruch 1 oder 2, worin Rb die Gruppe $-NR^2R^3$ und $R^2$ und $R^3$ je niederes Alkyl oder zusammen mit dem Stickstoffatom eine durch niederes Alkoxy oder niederes Alkoxyalkyl monosubstituierte 1-Azetidinyl- oder 1-Pyrrolidinylgruppe bedeuten.

**5.** Verbindungen nach einem der Ansprüche 1 bis 4, worin B zusammen mit dem mit $\alpha$ bezeichneten Kohlenstoffatom die Gruppe $>C_\alpha$-S-CH = CH-, $>C_\alpha$-CH = CH-CH = CH- oder $>C_\alpha$-CCl = CH-CH = CH- bedeutet.

**6.** N,N-Dimethyl-4,6-dihydro-4-oxo-3-phenylpyrido[2,1-a]isoindol-1-carboxamid.

**7.** 1-[(4,6-Dihydro-4-oxo-3-phenylpyrido[2,1-a]isoindol-1-yl)carbonyl]-3-methoxyazetidin.

**8.** 1-[(4,6-Dihydro-6-oxo-7-phenylthieno[2',3':3,4]pyrrolo[1,2-a]pyridin-9-yl)carbonyl]-3-methoxyazetidin.

**9.** (R)-1-[(4,6-Dihydro-6-oxo-7-phenylthieno[2',3':3,4]pyrrolo[1,2-a]pyridin-9-yl)carbonyl]-2-(methoxymethyl)pyrrolidin.

**10.** (S)-1-[(4,6-Dihydro-6-oxo-7-phenylthieno[2',3':3,4]pyrrolo[1,2-a]pyridin-9-yl)carbonyl]-3-methoxypyrrolidin.

**11.** 1-[(10-Chlor-4,6-dihydro-4-oxo-3-phenylpyrido[2,1-a]isoindol-1-yl)carbonyl]-3-methoxyazetidin.

**12.** Verbindungen nach einem der Ansprüche 1 bis 11 zur Anwendung als therapeutische Wirkstoffe.

**13.** Verbindungen nach einem der Ansprüche 1 bis 11 zur Anwendung als muskelrelaxierend, sedativhypnotisch, anxiolytisch und/oder antikonvulsiv wirksame Stoffe.

**14.** Verfahren zur Herstellung von Verbindungen nach einem der Ansprüche 1 bis 11 und von pharmazeutisch annehmbaren Säureadditionssalzen von Verbindungen der Formel I mit einem basischen Substituenten, dadurch gekennzeichnet, dass man

a) eine Carbonsäure der allgemeinen Formel

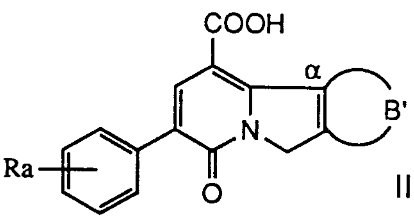

II

worin B' zusammen mit dem mit $\alpha$ bezeichneten Kohlenstoffatom die Gruppe $>C_\alpha$-S-CH = CH- oder $>C_\alpha$-CH = CH-CH = CH- bedeutet, und Ra die in Anspruch 1 angegebene Bedeutung besitzt, in Gegenwart eines Kondensationsmittels oder ein reaktives Derivat einer Carbonsäure der Formel II mit einer Verbindung der allgemeinen Formel

$R^1$-OH III

oder

$R^2R^3$NH IV

worin $R^1$, $R^2$ und $R^3$ die in Anspruch 1 angegebene Bedeutung besitzen, umsetzt, oder

b) eine Verbindung der allgemeinen Formel

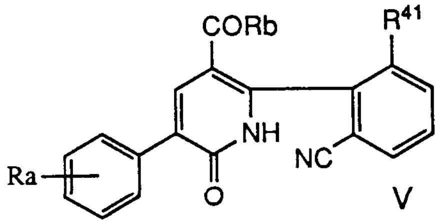

V

worin $R^{41}$ Chlor oder Fluor bedeutet, und Ra und Rb die in Anspruch 1 angegebene Bedeutung besitzen, mit Zink in Essigsäure behandelt, oder

c) eine Verbindung der allgemeinen Formel

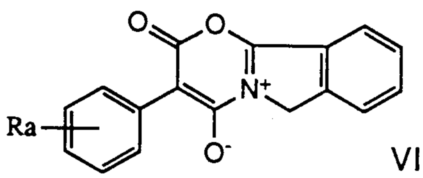

VI

worin Ra die in Anspruch 1 angegebene Bedeutung besitzt, mit einer Verbindung der allgemeinen Formel

$HC{\equiv}C\text{-}COOR^{11}$ VII

worin $R^{11}$ niederes Alkyl bedeutet, umsetzt, oder

d) in einer Verbindung der allgemeinen Formel

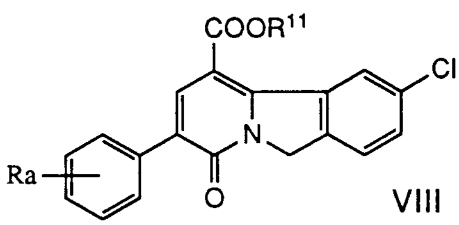

VIII

worin $R^{11}$ und Ra die in Anspruch 1 angegebene Bedeutung besitzen, das Chloratom durch Hydrogenolyse entfernt, oder

e) in einer Verbindung der allgemeinen Formel

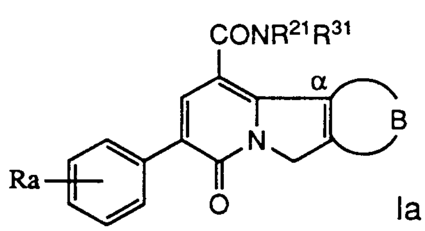

Ia

worin $R^{21}$ und $R^{31}$ zusammen mit dem Stickstoffatom eine durch niederes Alkoxy oder niederes Alkoxyalkyl substituierte 1-Azetidinyl-, 1-Pyrrolidinyl-, 1-Piperidinyl-, 4-Morpholinyl, 4-Thiomorpholinyl- oder 1-Piperazinylgruppe bedeuten, und Ra und B die in Anspruch 1 angegebene Bedeutung besitzen,
die Aethergruppe spaltet, oder
f) in einer Verbindung der allgemeinen Formel

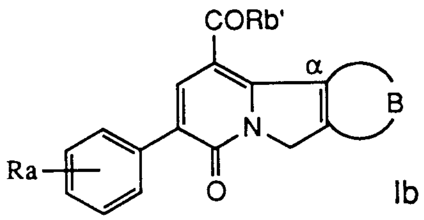

Ib

worin Rb' die Gruppe $-OR^{12}$ oder $-NR^{22}R^{32}$, $R^{12}$ und $R^{22}$ je eine durch niederes Alkoxycarbonylamino substituierte niedere Alkylgruppe und $R^{32}$ Wasserstoff oder niederes Alkyl bedeuten, und Ra und B die in Anspruch 1 angegebene Bedeutung besitzen,
die niedere Alkoxycarbonylgruppe abspaltet, und
g) erwünschtenfalls eine erhaltene Verbindung der Formel I mit einem basischen Substituenten in ein pharmazeutisch annehmbares Säureadditionssalz überführt.

**15.** Verbindungen der in Anspruch 14 definierten Formeln II, V, VI und VIII.

**16.** Arzneimittel enthaltend eine Verbindung nach einem der Ansprüche 1 bis 11 und ein therapeutisch inertes Trägermaterial.

**17.** Muskelrelaxierend, sedativ-hypnotisch, anxiolytisch und/oder antikonvulsiv wirksame Mittel enthaltend eine Verbindung nach einem der Ansprüche 1 bis 11 und ein therapeutisch inertes Trägermaterial.

**18.** Verwendung von Verbindungen nach einem der Ansprüche 1 bis 11 bei der Bekämpfung oder Verhütung von Krankheiten.

**19.** Verwendung von Verbindungen nach einem der Ansprüche 1 bis 11 bei der Bekämpfung oder Verhütung von Muskelspannungen, Spannungszuständen, Schlaflosigkeit, Angstzuständen und/oder Konvulsionen.

**20.** Verwendung von Verbindungen nach einem der Ansprüche 1 bis 11 zur Herstellung von muskelrelaxierend, sedativ-hypnotisch, anxiolytisch und/oder antikonvulsiv wirksamen Mitteln.

Europäisches Patentamt

**EUROPÄISCHER TEILRECHERCHENBERICHT,** der nach Regel 45 des Europäischen Patent-übereinkommens für das weitere Verfahren als europäischer Recherchenbericht gilt

Nummer der Anmeldung

EP 91113940.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP - A2 - 0 226 196 (HOFFMANN-LA ROCHE) * Zusammenfassung * -- | 1,15, 16,17, 20 | C 07 D 471/04<br>C 07 D 495/14<br>C 07 D 213/80<br>C 07 D 403/06<br>C 07 D 498/04<br>A 61 K 31/435<br>//(C 07 D 471/04<br>C 07 D 221:00<br>C 07 D 209:00)<br>(C 07 D 495/14<br>C 07 D 333:00<br>C 07 D 221:00<br>C 07 D 209:00) |
| A | CHEMICAL ABSTRACTS, Band 88, Nr. 23, 5. Juni 1978, Columbus, Ohio, USA SANO, TAKEHIRO et al. "Dioxopyrrolines. IX. A new route to 3,4-dihydro-2--pyridones from dioxooyrroline derivatives" Seite 570, Spalte 1, Zusammenfassung-Nr. 169 907d & Heterocycles 1978, 9(2), 161-8 ---- | 15 | |

RECHERCHIERTE SACHGEBIETE (Int. Cl.5)

C 07 D 471/00
C 07 D 495/00
C 07 D 213/00
C 07 D 403/00
C 07 D 498/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-17,20
Unvollständig recherchierte Patentansprüche: -
Nicht recherchierte Patentansprüche: 18,19 (Artikel 52(4) EPÜ;
Grund für die Beschränkung der Recherche: Verfahren zur therapeutischen Behandlung des menschlichen oder tierischen Körpers)

| Recherchenort | Abschlußdatum der Recherche | Prüfer |
|---|---|---|
| WIEN | 29-10-1991 | ONDER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82